## Europäisches Patentamt

## European Patent Office

## Office européen des brevets

(11) Veröffentlichungsnummer: **0 324 948**
**A2**

(12) **EUROPÄISCHE PATENTANMELDUNG**

(21) Anmeldenummer: **88120860.7**

(22) Anmeldetag: **14.12.88**

(51) Int. Cl.⁴: **A61B 17/22**

(30) Priorität: **21.01.88 DE 3801685**

(43) Veröffentlichungstag der Anmeldung:
**26.07.89 Patentblatt 89/30**

(84) Benannte Vertragsstaaten:
**CH DE ES IT LI NL**

(71) Anmelder: **DORNIER MEDIZINTECHNIK GMBH**
**Postfach 1128**
**D-8034 Germering 1(DE)**

(72) Erfinder: **Hepp, Wolfgang, Dr.-Ing.**
**Hardtstrasse 6**
**D-7997 Immenstaad(DE)**
Erfinder: **Denk, Roland, Dipl.-Phys.**
**Rankestrasse 11**
**D-8000 München 40(DE)**
Erfinder: **Forssmann, Bernd, Dr.rer.nat.**
**Eisenbahnstrasse 50**
**D-8034 Germering(DE)**
Erfinder: **Eizenhöfer, Harald, Dipl.-Phys.**
**Oberafferbacher Strasse 2**
**D-8752 Johannesberg(DE)**

(74) Vertreter: **Landsmann, Ralf, Dipl.-Ing.**
**DORNIER GMBH - Patentabteilung - Kleeweg 3**
**D-7990 Friedrichshafen 1(DE)**

(54) **Vorrichtung zur Steinzerkleinerung.**

(57) Vorrichtung und Verfahren zur extrakorporalen Zerkleinerung von Konkrementen in Körpern von Lebewesen durch Beschallung der Konkremente mit einer Pulsschall-Hochleistungsquelle (1), die - bevorzugt fokussierte - Druckwellenimpulse aussendet, deren Repetitionsfrequenz so durchgesteuert werden kann, dass sie mit einer der Resonanzfrequenzen der Konkremente (2) übereinstimmt.

Fig. 5

## Vorrichtung zur Steinzerkleinerung

Die Erfindung betrifft eine Vorrichtung zur Steinzerkleinerung nach dem Oberbegriff des Anspruchs 1.

Die extrakorporale Zerkleinerung von Konkrementen (z.B. Nieren- oder Gallensteinen) im menschlichen Körper wird bisher mit Stosswellen durchgeführt, die mit niedriger Wiederholrate auf den Stein gerichtet werden. Eine Addition der Energie im Stein findet nicht statt, da infolge der hohen Dämpfung die Schwingung im Stein abgeklungen ist, bevor die nächste Stosswelle eintrifft.

Aus der DE-PS 32 40 691 ist eine Vorrichtung bekannt, die solche Pulse in Pulsfolgen aufteilt, die so dicht hintereinander folgen, dass sie gleichzeitig im Konkrement wirken. Allerdings lassen sich damit nur wenige Pulsfolgen erzeugen, da deren Amplitude schnell abnimmt.

Aufgabe der Erfindung ist es, eine Vorrichtung und ein Verfahren zu schaffen, mit dem Konkremente effektiv nicht invasiv zerkleinert werden können.

Diese Aufgabe wird erfindungsgemäss gelöst von Vorrichtungen und Verfahren mit den Merkmalen der Ansprüche.

Die Erfindung benutzt das Prinzip, dass jeder feste Körper zu Volumenschwingungen verschiedenster Form und räumlich sowie zeitlich veränderlicher Ausprägung angeregt werden kann. Diese Schwingungen schaukeln sich besonders stark auf, wenn die Anregung mit einer der (vielen) Resonanzfrequenzen des Festkörpers (Konkrements) übereinstimmt.

Eine Pulsschall-Hochleistungsquelle, vorteilhafterweise fokussierend, sendet gepulste Druckwellenimpulse aus, deren Repetitionsfrequenz so durchgesteuert wird, dass sie mit einer dieser Resonanzfrequenzen übereinstimmt. Dadurch kommt es zu einer Aufschaukelung der dann bevorzugten Schwingung, die schliesslich bis zur Rißbildung im Steinmaterial führt.

Durch kontinuierliches Sweepen werden Konkremente und Konkrementreste verschiedener Größe und Zusammensetzung in Resonanzfrequenz versetzt und zerkleinert, so dass schließlich nur Konkrementreste unter einer bestimmten Größe $d_{min}$ übrigbleiben. Die Größe hängt in folgender Weise mit der Schallgeschwindigkeit c des Materials und der Repetitionsfrequenz fr zusammen:

$$d_{min} \sim \frac{c}{fr}$$

Die Hochleistungsschallquelle ist mechanisch und/oder elektrisch stark gedämpft, so dass sie möglichst kurze Pulse aussendet. Typische Resonanzfrequenzen der Schallquelle liegen zwischen 0,1 und 10 MHz, typische Repetitionsfrequenzen zwischen 1 KHz und 1 MHz.

Die Schallpulse können sich auf ihrem Weg zum Konkrement zur Stosswelle aufsteilen. Die negativen Anteile der Pulse werden durch geeignete Maßnahmen unterdrückt, so dass eine Schädigung des dazwischen liegenden Gewebes vermieden wird.

Bevorzugt sind piezoelektrische Schallquellen dazu geeignet, bei denen eine Vielzahl von Piezoelementen auf einem kugelkalottenförmigen Träger angeordnet sind, was eine mechanische Fokussierung ergibt. Die erfindungsgemäß angegebenen Frequenzen ermöglichen einen raschen Zerfall der Konkremente in abgangsfähige Bruchstücke.

Geht man davon aus, daß Konkremente bis auf eine Reststeingröße von maximal 2 mm zerkleinert werden sollen, ergibt sich eine maximale Repetitionsfrequenz von 1 MHz. Vorteilhaft wird deshalb die Repetitionsfrequenz zwischen 100 KHz und 1 MHz in oben beschriebener Weise durchgestimmt.

Es kann vorteilhaft sein, die untere und die obere Repetionsfrequenzgrenze mit fortschreitender Zerkleinerung zu höheren Frequenzen zu verschieben.

Zur Vermeidung von Gewebeschäden durch Kavitation und Wärmeentwicklung wurden unipolare Druckpulse verwendet, wie sie in bekannter Weise, z.B. durch piezoelektrische Wandler, die elektrisch oder mechanisch entsprechend bedämpft sind, fokussiert abgestra werden. Wegen der hohen Repetitionsrate eignen sich Wandler, die auf dem piezoelektrischen, magnetostriktiven oder elektromagnetischen Prinzip beruhen. Je nach Lage, Größe und Konsistenz des Steines können Druckamplituden zwischen 0,1 und 50 MPa notwendig werden. Es ist für die Zertrümmerungswirkung unter Umständen vorteilhaft, aber nicht notwendig, daß sich die Druckpulse auf ihrem Weg von der Pulsquelle zum Stein aufsteilen.

Die Erfindung wird anhand von 7 Fig. näher erläutert:
Es zeigen:

Fig. 1 mögliche Eigenschwingungen eines Konkrements

Fig. 2 das Schwingungsverhalten eines Konkrements

Fig. 3 den zeitlichen Verlauf von Ausgangspulsen einer erfindungsgemäßen Vorrichtung

Fig. 4 den zeitlichen Verlauf von Repetitionsfrequenzen

Fig. 5 ein Blockschaltbild einer erfindungsgemäßen Vorrichtung

Fig. 6 und 7 zwei mögliche Schaltungen zur Pulsformung.

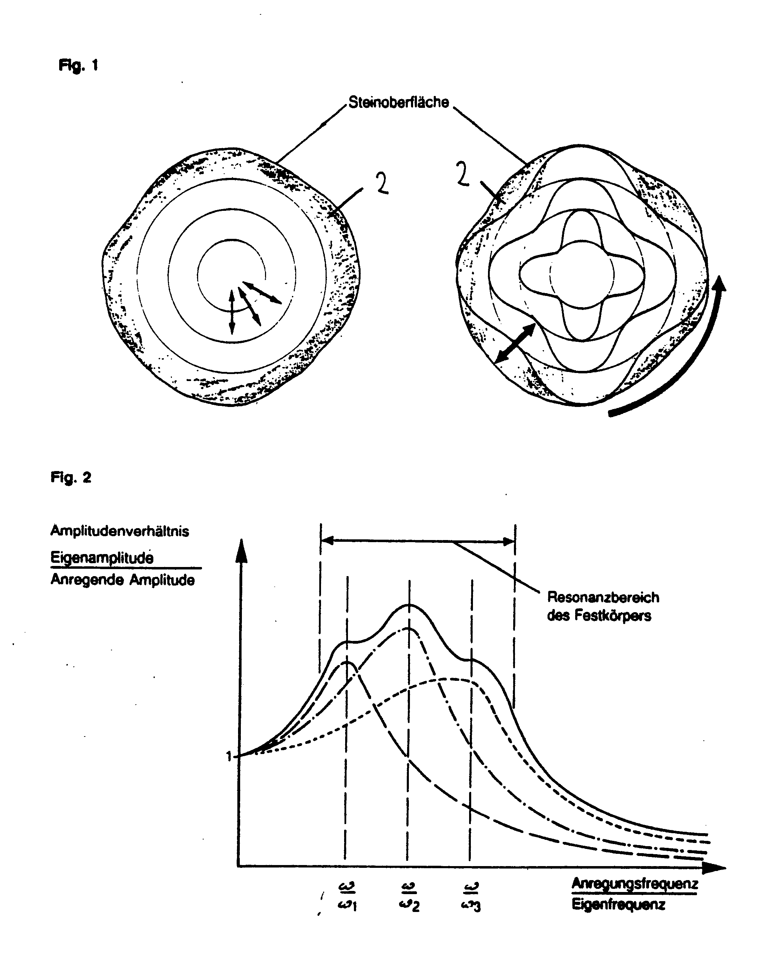

Fig. 1 zeigt schematisch mögliche Eigenschwingungen in einem Konkrement 2 (Nierenstein oder Gallenstein). Links eine symmetrische, zeitlich konstante Eigenschwingung mit stationären Knotenflächen, rechts ein symmetrische, zeitlich nicht konstante Eigenschwingung mit umlaufenden Knotenflächen.

Fig. 2 zeigt mit der geschlossenen Kurve das Schwingungsverhalten eines Konkrements 2 in Abhängigkeit von der Anregungsfrequenz. Aufgetragen ist das Amplitudenverhältnis ( = Verhältnis der Schwingungsamplitude (Eigenamplitude) zur anregenden Amplitude) über die Anregungsfrequenz. Die Kurve beginnt bei niedriger Frequenz mit dem Amplitudenverhältnis 1, d.h. der Stein 2 schwingt mit der Amplitude, mit der er angeregt wird. Mit steigender Frequenz wächst das Amplitudenverhältnis über 1 und erreicht im sogenannten Resonanzbereich des Festkörpers ein oder mehrere (hier 3) Maxima. Diese liegen bei den Eigenfrequenzen $\omega_1$ $\omega_2$ und $\omega_3$. Bei weiter steigender Anregungsfrequenz fällt die Amplitude der Steinschwingung stark ab. Die zu hohen Frequenzen können den Stein nur mehr zu kleinen Schwingungen anregen. Aus der Kurve wird deutlich, daß es Frequenzen gibt, auf die der Stein besonders gut anspricht (Resonanzfrequenzen $\omega_1$, $\omega_2$, $\omega_3$). Regt man den Stein mit diesen Frequenzen an, nimmt er besonders viel Energie auf und schwingt heftig. Dabei kann er leicht reissen oder springen - was Ziel der Erfindung ist. Gestrichelt sind 3 Dämpfungskurven gezeigt, die den 3 Eigenresonanzen entsprechen.

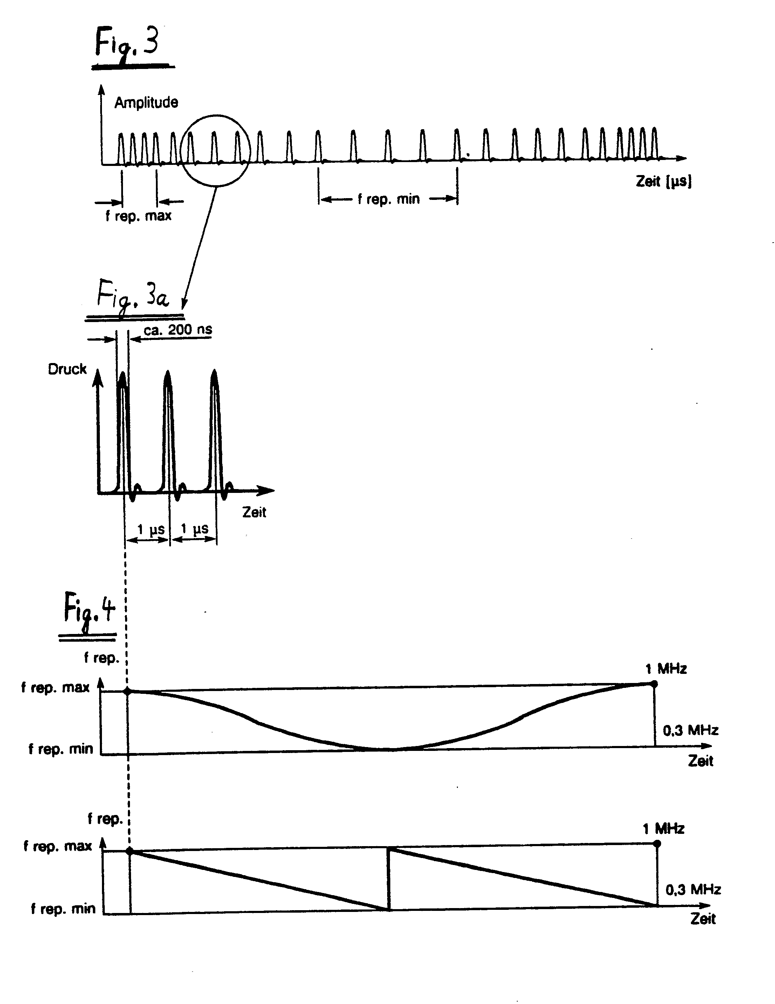

Fig. 3 zeigt den zeitlichen Verlauf von Druckpulsen, wie sie von einer erfindungsgemäßen Vorrichtung ausgesandt werden. Die Repetitionsfrequenz $f_{rep}$ variiert periodisch, d.h., zu bestimmten Zeiten folgen viele Pulse kurz hintereinander (maximale Repetitionsfrequenz $f_{rep}$ max) zu anderen Zeiten folgen die Pulse in großen Zeiträumen (minimale Repetitionsfrequenz $f_{rep}$ min). Die wechselnden Pulsfolgen entsprechen einem Durchfahren des Fre quenzbereichs, innerhalb dessen die Eigenfrequenzen des Konkrements 2 liegen. Durch die Anregung wird die Steinzerkleinerung erleichtert.

Fig. 3 a zeigt einen Ausschnitt aus Fig. 3. Die Druckpulse sind sehr steil, haben nur kleine Nachschwinger (starke Dämpfung), sind sehr kurz, ca. 200 ns, und folgen hier mit ungefähr 1 $\mu$sec Abstand ($f_{rep}$ = 1 MHz).

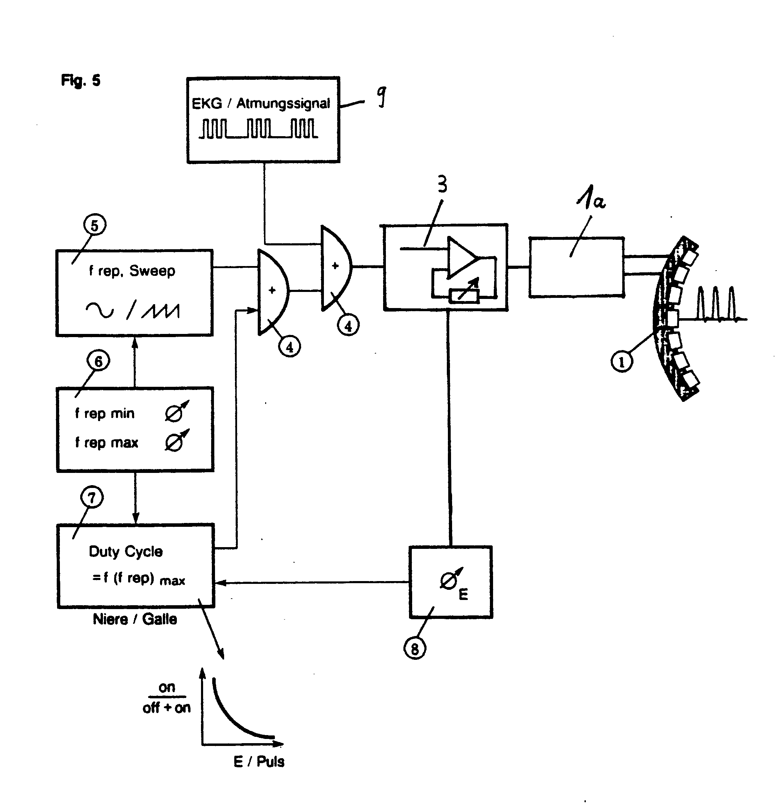

Fig. 4 zeigt, wie die Repetitionsfrequenzen zeitlich verlaufen können. Oben ist ein kontinuierliches, sinusförmiges Durchstimmen (Sweepen) gezeigt. Die Repetitionsfrequenz $f_{rep}$ variiert kontinuierlich zwischen 1 MHz und 0,3 MHz, entsprechend Fig. 3. Unten ist eine andere Möglichkeit des Durchstimmens gezeigt, bei der die Änderung der Repetitionsfrequenz sägezahnartig erfolgt. $F_{rep}$ fällt hier linear von 1 MHz auf 0,3 MHz, steigt sehr schnell wieder auf 1 MHz um wieder linear auf 0,3 MHz zu fallen.

Fig. 5 zeigt eine vorteilhafte technische Lösung für die Erzeugung von Pulsfolgen mit den oben beschriebenen Eigenschaften im Blockschaltbild.

Fig. 5 zeigt:
- eine Pulsschall-Hochleistungs-Quelle 1, z.B. eine piezokeramischen Wandler mit Einzelelementen, die auf einer Kugelkalotte angeordnet sind.
- einen Pulsgenerator 1 a zur Ansteuerung der Pulsschall-Hochleistungs-Quelle 1
- einen einstellbaren Verstärker 3
- zwei "Und" Gatter 4
- einen Repetitions-Frequenz-Generator 5
- einen Sweep-Generator 6 zur Vorwahl der oberen und unteren Repetitionsfrequenzen
- den Duty-Cycle-Regler 7 zum Einstellen der Pulszahl in Abhängigkeit von Gewebe und Pulsenergie
- die Energievorwahl 8
- eine EKG/Atemsynchronisationseinheit 9, die die Pulsauslösung in bekannter Weise mit der Atmung oder dem EKG des Patienten triggert. Damit können Interaktionen mit dem Gewebe durch Kavitation oder mit dem Herzen vermieden werden. Die Pulse werden in Form von Salven (Bursts) zusammengefaßt und getriggert ausgelöst. In bekannter Weise sind sie hier atemphasengesteuert, um die Trefferquote zu erhöhen.

Eine entsprechend bedämpfte Pulsschall-Hochleistungs-Quelle 1 (Stoßquelle) wird von einem Pulsgenerator 1 a angesteuert, der seinerseits von einer Energieversorgung 3 gespeist und von einem Und-Gatter 4 getriggert wird (Triggereingang bei 3). Dieses Und-Gatter 4 gibt die vom Repetitionspulsgenerator 5 kommenden Signale frei, wenn auch von der EKG/Atemsynchronisationseinheit 9 Signale ankommen.

Der Repetitionsgenerator 5 wird seinerseits von einem Sweep-Generator 6 angesteuert, der mit festen, per Hand wählbaren oder automatisch aufeinander folgenden Programmen die Repetitionsfrequenz steuert, wie es z. B. in Fig. 4 gezeigt ist.

Durch die Steuerung der Versorungsspannung in Abhängigkeit des Programmes des Sweep-Generators 6 kann die Amplitude der abgegebenen Pulse so gesteuert werden, daß unabhängig von der Repetitionsfrequenz eine wählbare akustische Leistung der Pulsschallquelle 1 nicht überschritten wird.

Die Repetitionssalven (bursts) können auch so fraktioniert sein, daß nach einer bestimmten Dauer, die kleiner ist als die Inkubationszeit von schädigenden Kavitationsblasen im Gewebe, die Salve abgebrochen und erst nach einer bestimmten "Aus"- Zeit wieder aufgenommen wird. Erreicht wird dies durch einen weiteren Triggergenerator (Duty Cyle Regler 7), der den Duty Cyle des Repetitionsgenerators 5 gewebeabhängig (z.B. für die Nieren und die Leber) steuert.

Infolge der hohen Pulsfrequenz eignen sich thyristor oder thyratrongesteuerte Hochleistungspulsgeneratoren.

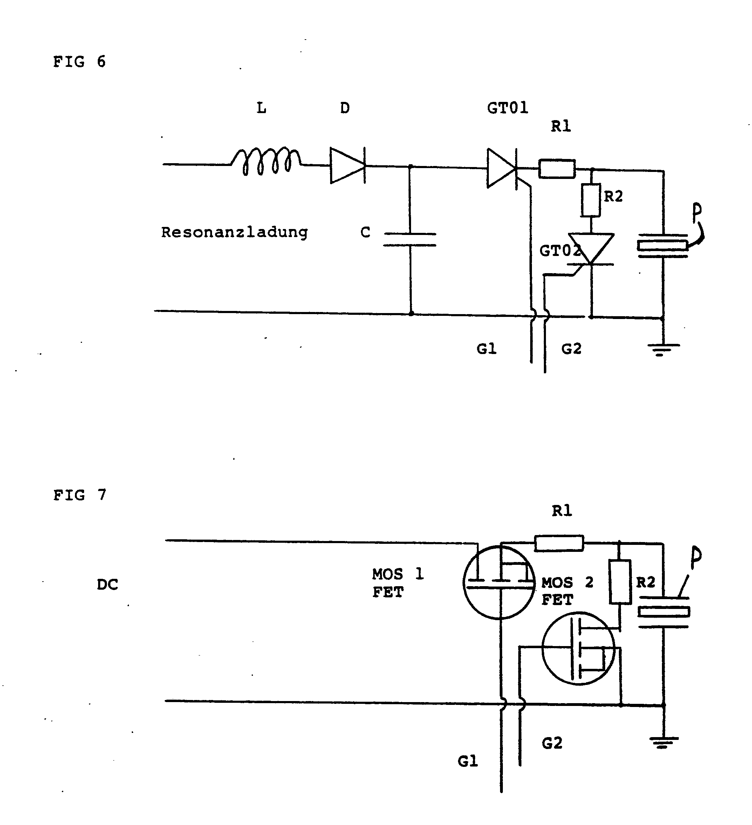

Fig. 6 zeigt eine Schaltungsmöglichkeit zur Realisierung der Baugruppen 1 a und 3 der Figur 5. Die Schaltung ist hier mit zwei Gate-turn-off Thyristoren (GTO 1 und GTO 2) realisiert. Gezeigt sind die Induktivität L, eine Diode D, der Ladungsspeicher C, zwei Thyristoren GTO 1 und GTO 2, zwei Widerstände R 1 und R 2 und die Piezokeramik P der Pulsschall-Hochleistungsquelle 1. Die gezeigte Schaltung erlaubt hohe Betriebsspannungen (einige kV) und mittlere Wiederholraten ($\leq$ 50 kH).

Fig. 7 zeigt eine Ausführung, die der in Fig. 6 gezeigten entspricht, jedoch mit MOSFET anstelle der Thyristoren. Damit sind niedrige Betriebsspan-nungen ($\leq$ 100 V) und hohe Wiederholraten (einigen 100 kHz bis MHz) möglich.

Für die beiden in den Figuren 6 und 7 gezeigten Schaltung gilt: Die Systeme können wegen thermischer Belastung nur im Burst-Mode betrieben werden. Die Schalter GTO 2 bzw. MOSFET 2 dienen zur schnellen Entladung der Piezos P. Dieser Entladevorgang kann zur Erzeugung eines nutzbaren akustischen Impulses genutzt werden.

## Ansprüche

1. Vorrichtung zur extrakorporalen Zerkleinerung von Konkrementen in Körpern von Lebewesen, **gekennzeichnet** durch eine Pulsschall-Hochleistungsquelle (1) die - bevorzugt fokussierte - Druckwellenimpulse aussendet, deren Repetitionsfrequenz so durchgesteuert werden kann, dass sie mit einer der Resonanzfrequenzen der Konkremente (2) übereinstimmt.

2. Vorrichtung nach Anspruch 1, gekennzeichnet durch eine piezoelektrische Pulsschall-Hochleistungsquelle (1) mit Resonanzfrequenzen zwischen 0,1 und 10 MHz und typischen Repetitionsfrequenzen zwischen 1 KHz und 1 MHz.

3. Verfahren zur extrakorporalen Zerkleinerung von Konkrementen in Körpern von Lebewesen, **gekennzeichnet** durch eine Beschallung der Konkremente mit einer Pulsschall-Hochleistungsquelle (1), die - bevorzugt fokussierte - Druckwellenimpulse aussendet, deren Repetitionsfrequenz so durchgesteuert werden kann, dass sie mit einer der Reso-nanzfrequenzen der Konkremente (2) überein-stimmt.

4. Verfahren nach Anspruch 3, dadurch ge-kennzeichnet, dass die Resonanzfrequenzen zwi-schen 0,1 und 10 MHz und die Repetitionsfrequenzen zwischen 1 KHz und 1 MHz liegen.

**Fig. 1**

Steinoberfläche

**Fig. 2**

Amplitudenverhältnis

$$\frac{\text{Eigenamplitude}}{\text{Anregende Amplitude}}$$

Resonanzbereich des Festkörpers

$$\frac{\text{Anregungsfrequenz}}{\text{Eigenfrequenz}}$$

Fig. 3

Fig. 3a

Fig. 4

EP 0 324 948 A2

**Fig. 5**

EKG / Atmungssignal — 9

① f rep, Sweep    $\sim$ / $\Lambda\Lambda\Lambda$

⑥ f rep min    f rep max

⑦ Duty Cycle    = f (f rep) max    Niere / Galle

④ ④    ③    1a    ①

⑧ $\varnothing$ E

$\frac{on}{off + on}$

E / Puls

FIG 6

L  D  GT01

R1

Resonanzladung

C

R2

GT02

G1  G2

P

FIG 7

R1

DC

MOS 1 FET

MOS 2 FET

R2

P

G1  G2